(19) Europäisches Patentamt
European Patent Office
Office européen des brevets

(11) Veröffentlichungsnummer: **0 246 475 B1**

# (12) EUROPÄISCHE PATENTSCHRIFT

(45) Veröffentlichungstag der Patentschrift: **29.01.92**

(51) Int. Cl.$^5$: **C07C 45/50**

(21) Anmeldenummer: **87106362.4**

(22) Anmeldetag: **30.04.87**

Die Akte enthält technische Angaben, die nach dem Eingang der Anmeldung eingereicht wurden und die nicht in dieser Patentschrift enthalten sind.

## (54) Verfahren zur Herstellung von Aldehyden.

(30) Priorität: **13.05.86 DE 3616057**

(43) Veröffentlichungstag der Anmeldung:
**25.11.87 Patentblatt 87/48**

(45) Bekanntmachung des Hinweises auf die Patenterteilung:
**29.01.92 Patentblatt 92/05**

(84) Benannte Vertragsstaaten:
**AT BE CH DE ES FR GB IT LI LU NL SE**

(56) Entgegenhaltungen:
**EP-A- 163 234**
**EP-A- 0 008 459**
**EP-A- 0 173 219**
**GB-A- 2 075 857**

(73) Patentinhaber: **HOECHST AKTIENGESELLSCHAFT**
**Postfach 80 03 20**
**W-6230 Frankfurt am Main 80(DE)**

(72) Erfinder: **Bach, Hanswilhelm, Dr.Dipl.-Chem.**
**Alleestrasse 56**
**W-4100 Duisburg 11(DE)**
Erfinder: **Bahrmann, Helmut, Dr.Dipl.-Chem.**
**Rohstrasse 48**
**W-4236 Hamminkeln-Brünen(DE)**
Erfinder: **Cornils, Boy, Dr. Dipl.-Chem.**
**Kirschgartenstrasse 6**
**W-6238 Hofheim(DE)**
Erfinder: **Konkol, Werner, Dr. Dipl.-Chem.**
**Lützowstrasse 40a**
**W-4200 Oberhausen 11(DE)**
Erfinder: **Wiebus, Ernst**
**Ferdinandstrasse 77**
**W-4200 Oberhausen 11(DE)**

EP 0 246 475 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von Aldehyden durch Umsetzung olefinisch ungesättigter Verbindungen in wäßrigem Medium und in Gegenwart wasserlöslicher Rhodium-Komplexverbindungen als Katalysator. Ziel der neuen Arbeitsweise ist, durch Präformierung des Katalysators die Reaktionszeit in der Anfangsphase der Umsetzung zu verkürzen und den Austrag von Edelmetall in diesem Reaktionsabschnitt zu unterbinden.

Die Herstellung von Aldehyden und Alkoholen durch Umsetzung von Olefinen mit Kohlenmonoxid und Wasserstoff ist bekannt. Die Reaktion wird durch Hydridometallcarbonyle, insbesondere von Metallen der 8. Nebengruppe des Periodensystems katalysiert. Während das klassische Verfahren in seinen verschiedenen technischen Ausgestaltungen Kobaltkatalysatoren verwendet, gewinnen in letzter Zeit Rhodiumkatalysatoren zunehmend Bedeutung. Im Vergleich zu Kobalt ermöglicht Rhodium als Katalysatorbestandteil die Umsetzung bei niedrigem Druck durchzuführen; darüber hinaus werden vorzugsweise geradkettige n-Aldehyde und nur in untergeordnetem Maße iso-Aldehyde gebildet. Schließlich ist auch die als Nebenreaktion mögliche Hydrierung der Olefine zu gesättigten Kohlenwasserstoffen bei Anwendung von Rhodiumkatalysatoren deutlich niedriger als bei Anwendung von Kobaltkatalysatoren.

Bei den in der Technik eingeführten Verfahren werden als Rhodiumkatalysatoren modifizierte Hydridorhodiumcarbonyle eingesetzt` d.h. Verbindungen, die neben Rhodium, Wasserstoff und Kohlenmonoxid noch mindestens einen weiteren Liganden enthalten. Solche Liganden sind organische Verbindungen eines Elementes der Gruppe Va des Periodensystems sowie Ester z.B. der phosphorigen oder der arsenigen Säure. Besonders bewährt haben sich tertiäre Phosphine oder Phosphite. Üblicherweise werden sie im Überschuß eingesetzt und bilden dann einen Teil des Reaktionsmediums.

Unter den Hydroformylierungsverfahren, die mit modifizierten Hydridorhodiumcarbonylen als Katalysatoren arbeiten, weist das in der DE-PS 26 27 354 beschriebene Verfahren eine Besonderheit auf. Die Umsetzung von Olefin, Kohlenmonoxid und Wasserstoff erfolgt bei ihm in flüssiger Phase in Gegenwart von Wasser und wasserlöslichen Rhodium-Komplexverbindungen. Die Löslichkeit der Rhodium-Komplexverbindungen wird durch Verwendung sulfonierter Triarylphosphine als Komplexbestandteil erreicht. Diese Arbeitsweise hat eine Reihe bemerkenswerter Vorteile. Sie erlaubt insbesondere eine sehr einfache Trennung von Reaktionsprodukt und Katalysator und stellt eine annähernd vollständige Rückgewinnung des Rhodiums sicher. Die Abscheidung des Katalysators vom Reaktionsprodukt erfolgt einfach durch Trennung von wäßriger und organischer Phase, d.h. ohne Destillation und damit ohne thermische Belastung der gebildeten Aldehyde und Alkohole. Aufgrund der überaus geringen Löslichkeit des Katalysators in Aldehyd und Alkohol wird mit dem Reaktionsprodukt auch kaum Edelmetall ausgetragen.

Das Katalysatorsystem wird entweder gesondert hergestellt und dann in die Reaktionszone eingebracht oder in situ gebildet. Der erste Weg erfordert eigens Apparaturen zur Umsetzung der Ausgangssubstanzen Rhodium oder Rhodiumverbindung, wasserlösliches Phosphin, Kohlenmonoxid und Wasserstoff. Überdies muß die wäßrige Lösung des Reaktionsproduktes in den Reaktor überführt werden. Man bevorzugt daher den zweiten Weg, die in-situ-Herstellung des Katalysatorsystems im Hydroformylierungsreaktor. Hierbei geht man von Rhodium, einem Rhodiumoxid oder einem anorganischen Rhodiumsalz, dem wasserlöslichen Phosphin und Wasser als Lösungsmittel aus und behandelt das Gemisch mit Kohlenmonoxid und Wasserstoff bei Temperaturen und Drücken, wie sie für die Hydroformylierungsreaktion üblich sind. Ein Nachteil dieses Verfahrens ist, daß Rhodium und Rhodiumoxide aufgrund ihrer Unlöslichkeit in Wasser nur schwer reagieren und die wasserlöslichen anorganischen Rhodiumsalze wie Rhodiumchorid oder Rhodiumsulfat, korrodierend wirken und daher nur in Ausnahmefällen eingesetzt werden können. Statt wasserlöslicher Rhodiumsalze kann man auch solche einsetzen, die in organischen Lösungsmitteln löslich sind. Dann muß aber zu Beginn der Umsetzung, die zur Bildung des Katalysatorsystems führt und die mit der Hydroformylierungsreaktion einhergeht, mit Rhodiumverlusten gerechnet werden. Rhodium wird nämlich mit dem gebildeten Aldehyd zusammen so lange aus dem Reaktor ausgeschleust, wie noch Edelmetall im organischen Lösungsmittel vorhanden ist.

In Beispiel 1 der EP-A-163 234 wird im Zusammenhang mit Versuchen zur Hydroformylierung von n-Hexen-1 eine Art Katalysator-Präformierung beschrieben. Hierbei wird eine wässrige Lösung von Rhodiumacetat und wasserlöslichem Triarylphosphin bei 125 °C und unter einem Druck von 2,5 MPa mit Synthesegas behandelt. Ein Teil der umgesetzten Lösung, verbleibt im Reaktor und dient bei der sich anschließenden Hydroformylierung als Katalysator. Die Behandlung von Lösungen die nebeneinander Rhodiumsalz und Ligand enthalten, hat den Nachteil, daß als Reduktionsmittel gegenüber dem Rhodium fast ausschließlich der Ligand wirkt und nur im untergeordneten Maße das Synthesegas. Dadurch geht nicht nur wertvoller Ligand verloren, die Katalysatorlösung wird darüberhinaus durch inaktive Ballaststoffe, den oxidierten Ligand, verunreinigt. Um Fremdstoffe aus der Lösung fernzuhalten ist es überdies erforderlich, Rhodiumsalze in reiner Form einzusetzen.

Es bestand daher die Aufgabe ein Verfahren zu entwickeln, das es erlaubt, das aus Rhodium-Komplexverbindung und wasserlöslichem Phosphin bestehende Katalysatorsystems im Hydroformylierungsreaktor vorzubilden, ohne Nachteile durch Korrosion oder Edelmatellverlust oder unverhältnismäßig lange Reaktionszeiten zu erfahren.

Die Erfindung besteht in einem Verfahren zur Herstellung von Aldehyden durch Umsetzung olefinisch ungesättigter Verbindungen mit Kohlenmonoxid und Wasserstoff bei Temperaturen von 20 bis 150 °C und Drücken von 0,1 bis 20 MPa in flüssiger Phase in Gegenwart von Wasser und einer wasserlöslichen, Rhodium enthaltenden Komplexverbindung als Katalyator. Es ist dadurch gekennzeichnet, daß die Rhodium-Komplexverbindung im Hydroformylierungsreaktor vor Einsetzen der Hydroformylierungsreaktion aus dem in einem aliphatischen, cycloaliphatischen oder aromatischen Kohlenwasserstoff gelösten Rhodiumsalz einer Carbonsäure mit 2 bis 18 Kohlenstoffatomen durch Reaktion mit Kohlenmonoxid und Wasserstoff bei Drücken von 0,1 bis 1,8 MPa und Temperaturen von 50 bis 100 °C vorgebildet wird, wobei die Reaktion in Gegenwart einer wäßrigen Lösung eines wasserlöslichen Triarylphosphins erfolgt oder diese wäßrige Lösung nach der Reaktion der zuvor hergestellten Rhodium-Komplexverbindung zugesetzt wird, und danach das Olefin zugeführt wird.

Überraschenderweise hat sich gezeigt, daß durch Einhaltung der erfindungsgemäßen Reaktionsbedingungen das aktive Katalysatorsystem innerhalb weniger Stunden gebildet wird. Obwohl Zentralatom und Liganden in verschiedenen Phasen eines heterogenen Zweiphasensystems vorliegen, erfolgt die Reduktion des Rhodiums und sein Übergang aus dem organischen Lösungsmittel in die Wasserphase mit ausreichender Geschwindigkeit.

Als Reduktionsmittel gegenüber dem Rhodium wirken das Kohlenmonoxid-Wasserstoff-Gemisch, insbesondere aber die in der wäßrigen Phase gelösten wasserlöslichen Arylphosphine. Sie werden zu Verbindungen des fünfbindigen Phosphors oxidiert, die mit Rhodium keine katalytisch aktive Komplexverbindung bilden und als Liganden verlorengehen. Durch die Trennung von organischer, Rhodium enthaltender Phase und den Ligand enthaltender Wasserphase wird die Reduktion des Rhodiums durch den Ligand weitgehend ausgeschlossen. Dennoch kann es sich als zweckmäßig erweisen, die wäßrige Lösung des substituierten Arylphosphins erst nach der Umsetzung des Rhodiumsalzes mit Kohlenmonixid und Wasserstoff der organischen Phase zuzusetzen.

Zur Herstellung des Katalysatorsystems geht man von den Rhodiumsalzen organischer Säuren aus, die 2 bis 18 Kohlenstoffatome enthalten. Die Säuren können ein- oder mehrbasisch, geradkettig oder verzweigt sein. Sowohl Salze gesättigter oder ungesättigter aliphatischer Säuren als auch Salze aromatischer Säuren sind geeignet. Die Herstellung der Salze erfolgt z.B. durch Umsetzung wäßriger Rhodiumsalzlösungen wie Rhodium(III)-nitrat oder Rhodium(III)-sulfat mit den wäßrigen Lösungen von Salzen der organischen Säuren oder durch Umsetzung von Rhodiumoxid bzw. Rhodiumoxid-Hydraten mit den freien Säuren. Besonders geeignet für den Einsatz im erfindungsgemäßen Verfahren sind die Rhodiumsalze gesättigter Monocarbonsäuren mit 2 bis 10 Kohlenstoffatomen. Beispiele für diese Säuren sind Essigsäure, Propionsäure, n-Buttersäure, i-Buttersäure, Pentansäure, Hexansäure, 2-Ethylhexansäure. Besondere Reinigungsschritte im Anschluß an die Herstellung der Salze sind im allgemeinen nicht erforderlich. In den meisten Fällen kann das Umsetzungsprodukt unmittelbar in dem organischen Lösungsmittel aufgenommen werden, in dem anschließend die Umsetzung mit Kohlenmonoxid und Wasserstoff erfolgt.

Als organische Lösungsmittel werden aliphatische, cycloaliphatische oder aromatische Kohlenwasserstoffe eingesetzt. Besondere Anforderungen an die physikalischen Eigenschaften der Kohlenwasserstoffe werden nicht gestellt. Sie müssen aber selbstverständlich frei von solchen Verunreinigungen sein, die das katalytisch wirksame Rhodium desaktivieren. Die Konzentration des Rhodiums in dem Kohlenwasserstoff ist nicht kritisch. Zweckmäßig setzt man mäßig konzentrierte Lösungen ein, insbesondere solche, die mindestens 3000 mg Rhodium je l Lösung enthalten. Es ist nicht erforderlich, einheitliche Kohlenwasserstoffe einzusetzen. Auch Gemische verschiedener Kohlenwasserstoffe sind als Lösungsmittel für die Rhodiumsalze geeignet. Bewährt haben sich Pentan, Hexan, Benzinfraktionen des Rohöls, Toluol, Xylole.

Zur Umwandlung in die Vorstufe der katalytisch aktiven Form wird das in Kohlenwasserstoff gelöste Rhodiumsalz mit Kohlenmonoxid und Wasserstoff behandelt. Die Zusammensetzung des $CO/H_2$-Gemisches kann in weiten Bereichen variiert werden. Es ist möglich, sowohl kohlenmonoxidreiche als auch wasserstoffreiche Gemische zu verwenden.

Üblicherweise setzt man Mischungen ein, die Kohlenmonoxid und Wasserstoff etwa im Verhältnis 1 : 1 enthalten, d.h. eine Zusammensetzung aufweisen, wie sie auch in der anschließenden Hydroformylierung angewandt wird. Die Umsetzung der Rhodiumsalze erfolgt bei 50 bis 100 °C und Drücken von 0,1 bis 1,8 MPa. Bevorzugt werden 60 bis 90 °C und 0,2 bis 0,5 MPa, Bedingungen, die einen optimalen Verlauf der Reaktion sicherstellen. Als Umsetzungsprodukt bildet sich ein Rhodiumhydridocarbonyl. Entsprechend ihrer

Löslichkeit wandert die primär entstandene Rhodiumcarbonylverbindung in die wäßrige Lösung des wasserlöslichen Triarylphosphins und wird hier in die Rhodiumphosphin-Komplexverbindung umgewandelt.

Unter dem Begriff wasserlösliche Triarylphosphine werden Verbindungen verstanden, die auf Grund des Vorliegens einer oder mehrerer Sulfonat- oder Carboxylatgruppen im Molekül in Wasser löslich sind. Sie folgen der allgemeinen Formel

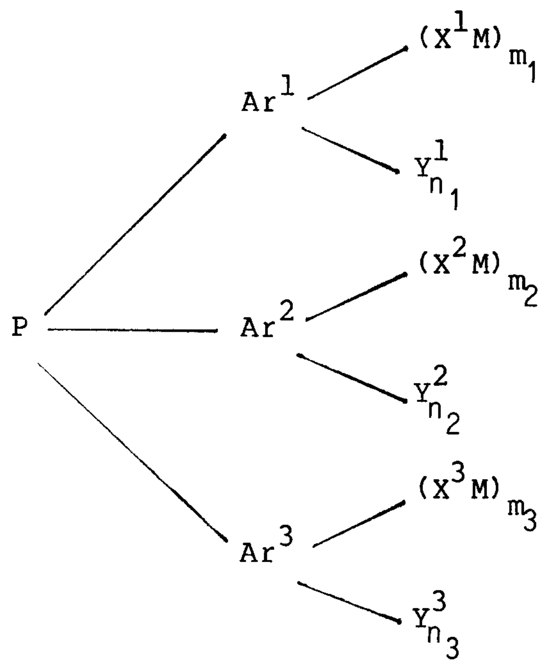

In ihr bedeuten $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$- oder $R^1R^2N$-Gruppe, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Carboxylat-($COO^-$-) und/oder ein Sulfonat-($SO_3^-$-)Rest, $m_1$, $m_2$, $m_3$ sind gleiche oder verschiedene ganze Zahlen von 0 bis 3, wobei mindestens eine Zahl $m_1$, $m_2$ oder $m_3$ gleich oder größer 1 ist; $n_1$, $n_2$, $n_3$ sind gleiche oder verschiedene ganze Zahlen von 0 bis 5. M ist ein Alkalimetallion, ein Äquivalent eines Erdalkalimetall- oder Zinkions, ein Ammonium- oder quaternäres Ammoniumion der allgemeinen Formel $N(R^3R^4R^5R^6)^+$, in der $R^3$, $R^4$, $R^5$, $R^6$ jeweils für eine geradkettige oder verzweigte Alkylgruppe mit bis zu 18 C-Atomen steht. Bewährt haben sich insbesondere quaternäre Ammoniumgruppen, in denen drei der Reste $R^3$, $R^4$, $R^5$, $R^6$ jeweils 1 bis 4 und der vierte Rest 1 bis 18 Kohlenstoffatome enthalten.

Bevorzugt werden wasserlösliche Triarylphosphine der vorstehend beschriebenen allgemeinen Formel, in der $Ar^1$, $Ar^2$, $Ar^3$ jeweils einen Phenylrest und $X^1$, $X^2$, $X^3$ jeweils einen Sulfonatrest oder einen Carboxylatrest bedeuten. Beispiele für Verbindungen der oben wiedergegebenen allgemeinen Formel sind Triphenylphosphin-tri-Na-trisulfonat, Triphenylphosphin-tri-(tetraalkylammonium)-trisulfonat, Triphenylphosphin-tri-Na-tricarboxylat.

Die sulfonierten oder carboxylierten Arylphosphine können als einheitliche Verbindungen eingesetzt werden. Es können aber auch Gemische aus Phosphinen verwendet werden, die unterschiedlich viele Sulfonsäuregruppen oder Carboxylatgruppen enthalten, also z.B. Gemische aus Triarylphosphintrisulfonaäuren und Triarylphosphindisulfonsäuren. Darüber hinaus müssen die Sulfonate oder Carboxylate nicht dasselbe Kation enthalten. Auch Gemische aus Salzen, die sich von unterschiedlichen Metallen ableiten und/oder Ammonium- und/oder quartäre Alkylammoniumionen enthalten, sind geeignet.

Die Konzentration der wasserlöslichen Triarylphosphine in der wäßrigen Lösung wird zweckmäßigerweise auf den Wert eingestellt, der für die sich anschließende Hydroformylierung erforderlich ist, d.h. auf etwa 25 bis 30 Gew.-%, bezogen auf die Lösung.

Wie bereits oben gesagt wurde, kann die Phosphin-Lösung der Rhodiumsalz-/Kohlenwasserstoff-Lösung zugesetzt werden. Um Phosphin-Verluste zu vermeiden, empfiehlt es sich aber häufig, zunächst die Rhodium-Carbonyl-Verbindung herzustellen und erst darauf die Phosphin-Lösung zuzugeben.

Der Fortgang der Umsetzung zwischen Phosphin und Rhodium kann aus der Abnahme der Rhodium-Konzentration in der organischen Phase bestimmt werden. Im allgemeinen ist die Umsetzung nach fünf bis

acht Stunden abgeschlossen. In der organischen Phase ist dann kein Rhodium mehr nachweisbar. Sobald dieser Zustand erreicht ist, können die für die Hydroformylierung erforderlichen Reaktionsbedingungen, nämlich Temperaturen von 20 bis 150 °C und Drücke von 0,1 bis 20 MPa eingestellt und dem Reaktor Olefin zugeführt werden.

Das nach der Präformierungsphase im Reaktor verbleibende organische Lösungsmittel wird zu Beginn der Umsetzung zwischen Olefin und Synthesegas mit dem gebildeten Aldehyd aus dem Reaktionssystem ausgeschleust und bei der Aufarbeitung des Reaktionsproduktes abgetrennt.

Das erfindungsgemäß Verfahren eignet sich ganz allgemein zur Hydroformylierung olefinisch ungesättigter Verbindungen. Besonders bewährt hat es sich bei der Umsetzung von Olefinen mit 2 bis 12 Kohlenstoffatomen. Diese Olefine können linear oder verzweigt sein und eine endständige oder innenständige Doppelbindung aufweisen. Beispiele für solche Olefine sind: Ethylen, Propylen, 1-Buten, 2-Buten, 1-Penten, 2-Methyl-1-buten, 4,4-Dimethyl-1-nonen, 1-Dodecen. Bevorzugt werden lineare Olefine mit 2 bis 8 Kohlenstoffatomen wie Ethylen, Propylen, 1-Buten, 1-Penten, 1-Hexen, 1-Hepten und 1-Octen.

Die wäßrige Katalysatorlösung enthält die wasserlöslichen Phosphine in einer Konzentration von 25 bis 30 Gew.-%, vorzugsweise 26 bis 28 Gew.-% und Rhodium in einer Konzentration von 450 bis 800 Gew.-ppm, vorzugsweise 500 bis 600 Gew.-ppm, jeweils bezogen auf die wäßrige Lösung.

Der Gesamtdruck von Wasserstoff und Kohlenmonoxid beträgt 1 bis 200 bar (100 bis $2 \cdot 10^4$ kPa), vorzugsweise 10 bis 100 bar ($1 \cdot 10^3$ bis $1 \cdot 10^4$ kPa). Die Zusammensetzung des Synthesegases, d.h. das Verhältnis von Kohlenmonoxid zu Wasserstoff kann in weiten Grenzen variiert werden. Im allgemeinen setzt man Synthesegas ein, in dem das Volumverhältnis von Kohlenmonoxid zu Wasserstoff 1 : 1 beträgt oder von diesem Wert nur wenig abweicht. Die Umsetzung erfolgt bei Temperaturen von 20 bis 150 °C, sie kann sowohl kontinuierlich als auch absatzweise durchgeführt werden.

Die nachfolgenden Beispiele erläutern die Erfindung.

Beispiel 1

In einem Autoklaven werden Lösungen von Triphenylphosphintrisulfonat in Wasser (etwa 30 Gew.-% Salz, bezogen auf die Lösung) und Rhodium-2-ethylhexanoat in Toluol (Rhodium-Gehalt etwa 10 g/l) unter Rühren bei etwa 80 °C und einem Druck von 0,4 MPa mit Synthesegas (CO : $H_2$ = 1 : 1) behandelt. Der Übergang des als Salz in Toluol gelösten Rhodiums in die Wasserphase wird durch regelmäßige Analyse des organischen Lösungsmittels überprüft. Nach etwa 5 Sunden ist in der organischen Phase Rhodium nicht mehr nachweisbar, der Rhodiumgehalt in der Wasserphase entspricht dann dem ursprünglich eingesetzten Rhodium.

Beispiel 2

In einem Autoklaven wird eine Lösung von Rhodiumhexanoat in Toluol (Rhodium-Gehalt 5 g/1) unter Rühren bei 70 °C und einem Druck von 0,7 MPa mit Synthesegas (CO : $H_2$ = 1 : 1) behandelt. Nach 3 Stunden wird der Lösung der Rhodiumverbindung eine solche Menge einer Lösung von Triphenylphosphintrisulfonat in Wasser (etwa 28 Gew.-% Salz, bezogen auf die Lösung) zugesetzt, daß ein P(III) : Rh-Verhältnis von ca. 100 : 1 resultiert. Man rührt eine weitere Stunde. Wie die Analyse zeigt, ist dann das Rhodium vollständig von der organischen in die Wasserphase übergegangen.

**Patentansprüche**

**1.** Verfahren zur Herstellung von Aldehyden durch Umsetzung olefinisch ungesättigter Verbindungen mit Kohlenmonoxid und Wasserstoff bei Temperaturen von 20 bis 150 °C und Drücken von 0,1 bis 20 MPa in flüssiger Phase in Gegenwart von Wasser und einer wasserlöslichen, Rhodium enthaltenden Komplexverbindung als Katalysator, dadurch gekennzeichnet, daß die Rhodium-Komplexverbindung im Hydroformylierungsreaktor vor Einsetzen der Hydroformylierungsreaktion aus dem in einem aliphatischen, cycloaliphatischen oder aromatischen Kohlenwasserstoff gelösten Rhodiumsalz einer Carbonsäure mit 2 bis 18 Kohlenstoffatomen durch Reaktion mit Kohlenmonoxid und Wasserstoff bei Drücken von 0,1 bis 1,8 MPa und Temperaturen von 50 bis 100 °C vorgebildet wird, wobei die Reaktion in Gegenwart einer wäßrigen Lösung eines wasserlöslichen Triarylphosphins erfolgt oder diese wäßrige Lösung nach der Reaktion der zuvor hergestellten Rhodium-Komplexverbindung zugesetzt wird, und danach das Olefin zugeführt wird.

**2.** Verfahren nach Anspruch 1, dadurch gekennzeichnet, daß das Rhodiumsalz sich von einer gesättigten

Monocarbonsäure mit 2 bis 10 Kohlenstoffatomen ableitet.

**3.** Verfahren nach Anspruch 2, dadurch gekennzeichnet, daß das Rhodiumsalz Rhodium(III)-2-ethylhexanoat ist.

**4.** Verfahren nach einem oder mehreren der Ansprüche bis 3, dadurch gekennzeichnet, daß das Rhodiumsalz in Pentan, Hexan, Benzin, Toluol oder Xylol gelöst ist.

**5.** Verfahren nach einem oder mehreren der Ansprüche 1 bis 4, dadurch gekennzeichnet, daß die Konzentration des Rhodiums in der Lösung mindestens 3000 mg/je 1 beträgt.

**6.** Verfahren nach einem oder mehreren der Ansprüche 1 bis 5, dadurch gekennzeichnet, daß die Umsetzung des Rhodiumsalzes mit Kohlenmonoxid und Wasserstoff bei 60 bis 90 °C und 0,2 bis 0,5 MPa erfolgt.

**7.** Verfahren nach einem oder mehreren der Ansprüche 1 bis 6, dadurch gekennzeichnet, daß wasserlösliche Triarylphosphine der allgemeinen Formel

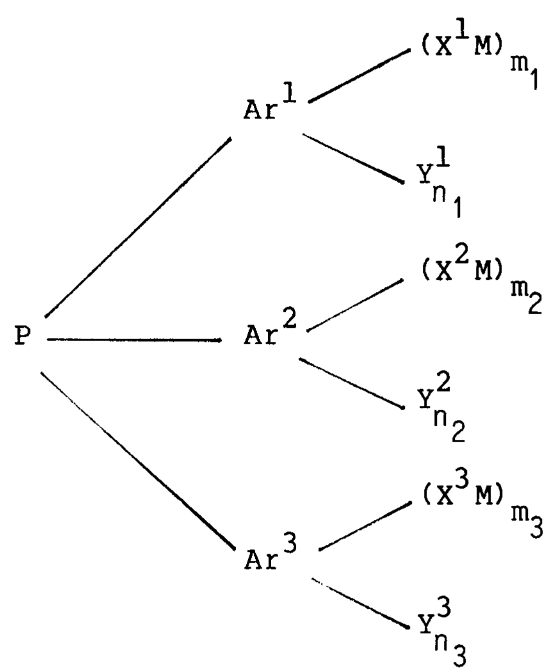

eingesetzt werden wobei $Ar^1$, $Ar^2$, $Ar^3$ jeweils eine Phenyl- oder Naphthylgruppe bedeuten, $Y^1$ $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$- oder $R^1R^2N$-Gruppe ist, in der $R^1$ und $R^2$ für jeweils eine geradkettige oder verzweigte Alkylgruppe mit 1 bis 4 C-Atomen stehen, $X^1$, $X^2$, $X^3$ jeweils ein Carboxylat-($COO^-$-)und/oder ein Sulfonat-($SO_3^-$-)Rest ist, $m_1$, $m_2$, $m_3$ gleiche oder verschiedene ganze Zahlen von 0 bis 3 sind, wobei mindestens eine Zahl $m_1$, $m_2$ oder $m_3$ gleich oder größer 1 ist $n_1$, $n_2$, $n_3$ gleiche oder verschiedene ganze Zahlen von 0 bis 5 sind und M für ein Alkalimetallion, ein Äquivalent eines Erdalkalimetall- oder Zinkions, ein Ammonium- oder quaternäres Ammoniumion der allgemeinen Formel $N(R^3R^4R^5R^6)^+$, in der $R^3$, $R^4$, $R^5$, $R^6$ jeweils für eine geradkettige oder verzweigte Alkylgruppe mit bis zu 18 C-Atomen steht, ist.

## Claims

**1.** A process for the preparation of aldehydes by the reaction of olefinically unsaturated compounds with carbon monoxide and hydrogen at temperatures of 20 to 150 °C and pressures of 0.1 to 20 MPa in the liquid phase in the presence of water and a water-soluble rhodium-containing complex compound as a catalyst, characterised in that before commencement of the hydroformylation reaction the rhodium

complex compound is preformed from the rhodium salt of a carboxylic acid with 2 to 18 carbon atoms dissolved in an aliphatic, cycloaliphatic or aromatic hydrocarbon by reaction with carbon monoxide and hydrogen at pressures of 0.1 to 1.8 MPa and temperatures of 50 to 100 °C, the reaction taking place in the presence of an aqueous solution of a water-soluble triarylphosphine or this aqueous solution being added after the reaction of the previously prepared rhodium complex compound.

**2.** A process according to claim 1, characterised in that the rhodium salt is derived from a saturated monocarboxylic acid with 2 to 10 carbon atoms.

**3.** A process according to claim 2, characterised in that the rhodium salt is rhodium(III)-2-ethylhexanoate.

**4.** A process according to one or several of the claims 1 to 3, characterised in that the rhodium salt is dissolved in pentane, hexane, gasoline, toluene or xylene.

**5.** A process according to one or several of the claims 1 to 4, characterised in that the concentration of rhodium in the solution is at least 3000 mg per litre.

**6.** A process according to one or several of the claims 1 to 5, characterised in that the rhodium salt is reacted with carbon monoxide and hydrogen at 60 to 90 °C and at 0.2 to 0.5 MPa.

**7.** A process according to one or several of the claims 1 to 6, characterised in that water-soluble triarylphosphines with the general formula

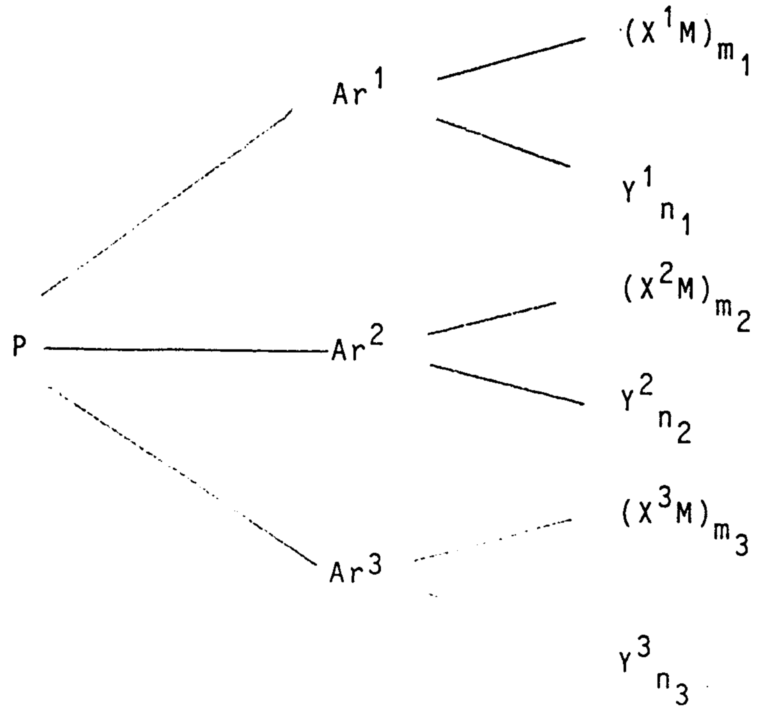

are employed, where $Ar^1$, $Ar^2$, $Ar^3$ each denote a phenyl or naphthyl groups $Y^1$, $Y^2$, $Y^3$ are each a straight-chain or branched alkyl group with 1 to 4 carbon atoms, an alkoxy group, a halogen atom, the OH, CN, $NO_2$ or $R^1R^2N$ group where $R^1$ and $R^2$ each stand for a straight-chain or branched alkyl group with 1 to 4 carbon atoms, $X^1$, $X^2$, $X^3$ being a carboxylate-($COO^-$-) group and/or a sulfonate-($SO_3^-$) group, $m_1$, $m_2$, $m_3$ are the same or different whole numbers from 0 to 3, at least one number $m_1$, $m_2$ or $m_3$ equalling or being larger than 1, $n_1$, $n_2$, $n_3$ being the same or different whole numbers from 0 to 5 and M standing for an alkali metal ion, an equivalent of an alkaline earth metal or zinc ion, an ammonium or quaternary ammonium ion with the general formula $N(R^3R^4R^5R^6)^+$, where $R^3$, $R^4$, $R^5$, $R^6$ are each a straight-chain or branched alkyl group with up to 18 carbon atoms.

## Revendications

1. Procédé pour la fabrication d'aldéhydes par réaction de composés à insaturation oléfinique avec le monoxyde de carbone et l'hydrogène à des températures de 20 à 150 °C et sous des pressions de 0,1 à 20 MPa en phase liquide en présence d'eau et d'un composé complexe soluble dans l'eau contenant du rhodium comme catalyseur, caractérisé en ce que le composé complexe de rhodium est préformé dans le réacteur d'hydroformylation avant le démarrage de la réaction d'hydroformylation à partir du sel de rhodium d'un acide carboxylique en $C_2$-$C_{18}$ dissous dans un hydrocarbure aliphatique, cycloaliphatique ou aromatique, par réaction avec le monoxyde de carbone et l'hydrogène sous des pressions de 0,1 à 1,8 MPa et à des températures de 50 à 100 °C, la réaction ayant lieu en présence d'une solution aqueuse d'une triarylphosphine soluble dans l'eau, ou bien cette solution aqueuse étant ajoutée après la réaction au composé complexe de rhodium préparé au préalable, et ensuite on envoie l'oléfine.

2. Procédé selon la revendication 1, caractérisé en ce que le sel de rhodium dérive d'un acide monocarboxylique saturé en $C_2$-$C_{10}$.

3. Procédé selon la revendicatin 2, caractérisé en ce que le sel de rhodium est le 2-éthylhexanoate de rhodium (III).

4. Procédé selon une ou plusieurs des revendications 1 à 3, caractérisé en ce que le sel de rhodium est dissous dans le pentane, l'hexane, l'essence, le toluène ou le xylène.

5. Procédé selon une ou plusieurs des revendications 1 à 4, caractérisé en ce que la concentration du rhodium dans la solution est d'au moins 3 000 mg/l.

6. Procédé selon une ou plusieurs des revendications 1 à 5, caractérisé en ce que la réaction du sel de rhodium avec le monoxyde de carbone et l'hydrogène s'effectue à 60-90 °C et sous 0,2-0,5 MPa.

7. Procédé selon une ou plusieurs des revendications 1 à 6, caractérisé en ce que l'on utilise des triarylphosphines solubles dans l'eau de formule générale :

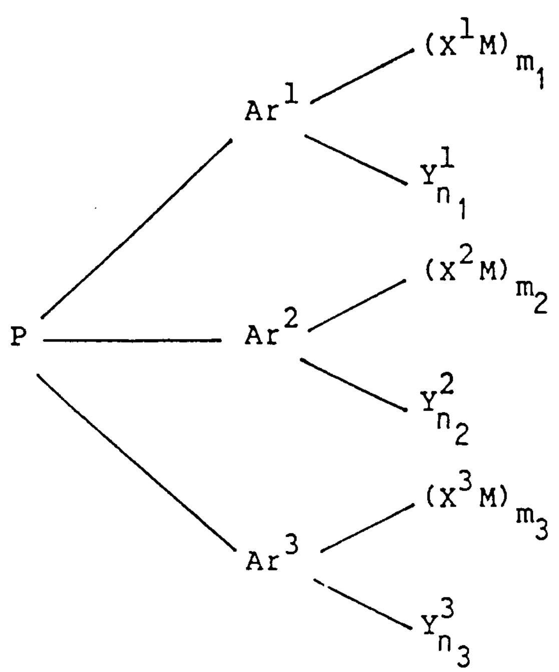

dans laquelle $Ar^1$, $Ar^2$, $Ar^3$ représentent chacun un groupe phényle ou un groupe naphtyle, $Y^1$, $Y^2$, $Y^3$ représentent chacun un groupe alkyle à chaîne droite ou ramifiée en $C_1$-$C_4$, un groupe alcoxy, un atome d'halogène, le groupe OH, le groupe CN, le groupe $NO_2$ ou le groupe $R^1R^2N$ dans lequel $R^1$ et $R^2$ représentent chacun un groupe alkyle à chaîne droite ou ramifiée en $C_1$-$C_4$, $X^1$, $X^2$, $X^3$ représentent

chacun un reste carboxylate ($COO^-$) et/ou un reste sulfonate ($SO_3^-$), $m_1$, $m_2$, $m_3$ sont des nombres entiers identiques ou différents de 0 à 3, au moins un nombre $m_1$, $m_2$ ou $m_3$ étant égal ou supérieur à 1, $n_1$, $n_2$, $n_3$ sont des nombres entiers identiques ou différents de 0 à 5 et M représente un ion de métal alcalin, un équivalent d'un ion de métal alcalino-terreux ou d'un ion zinc, un ion ammonium ou un ion ammonium quaternaire de formule générale $N(R^3R^4R^5R^6)^+$ dans laquelle $R^3$, $R^4$, $R^5$, $R^6$ représentent chacun un groupe alkyle à chaîne droite ou ramifiée jusqu'en $C_{18}$.